# EUROPEAN PATENT SPECIFICATION

(11) **EP 1 251 751 B1**
(45) Date of publication and mention of the grant of the patent: **04.05.2005**
(21) Application number: 01902895.0
(22) Date of filing: 24.01.2001
(51) Int. Cl.: A23L 3/18

(54) **DEVICE AND METHOD FOR KILLING MICROORGANISMS IN A FLUID FLOW OF PRODUCTS**
VORRICHTUNG UND VERFAHREN ZUM ABTOETEN VON MIKROORGANISMEN IN EINEM FLUESSIGSTROM VON PRODUKTEN
DISPOSITIF ET PROCEDE DESTINE A TUER DES MICRO-ORGANISMES DANS UN ECOULEMENT FLUIDE DE PRODUITS

(30) Priority: 31.01.2000 SE 0000273
(43) Date of publication of application: 30.10.2002
(73) Proprietor: Arom Pak International AB, 227 30 Lund (SE)
(72) Inventor: SJÖHOLM, Johan, S-224 78 Lund (SE); ERLANDSSON, Per-Olof, S-247 34 Södra Sandby (SE)
(74) Representative: Thylén, Eva Matilda
(86) International application number: PCT/SE2001/000122
(87) International publication number: WO 2001/056407

(56) References cited:
- EP-A1- 0 081 256
- EP-A2- 0 066 557
- EP-A2- 0 196 464
- DK-B- 160 177
- GB-A- 2 155 749
- US-A- 5 101 713
- US-A- 5 834 049

## Description

### Technical Field

The present invention relates to a device and a method for killing microorganisms in a fluid flow of products as well as use of such a device.

### Background of the Invention

When liquid products, such as liquid foods (e.g. milk) and different types of medicine, are sterilised the product may be heated to a sufficient temperature during a required period of time for killing microorganisms.

In a continuous process, this can be achieved by continuously supplying the product to a heating tank, from which the product is then continuously passed on. In this context the product is supplied to and discharged from the tank through an inlet and an outlet respectively, which are normally arranged at opposite ends of the tank. Accordingly, the flow through the tank will be random to some extent and, therefore, a situation might occur where the discharged product has not been in the tank long enough for adequate killing to take place. Moreover, the microorganisms have the ability to travel in the product in a way that differs from the flow pattern of the product. Thus even if the product remains in the tank during the required period of time this does not mean that the microorganisms will do so. Consequently, satisfactory killing of undesired microorganisms is not assured.

Admittedly, it is possible to make sure that a sufficient temperature is maintained in the product as it is discharged from the tank by insulating the discharge of the tank. In this way, it is possible to extend the time during which the product is heated. Again though, there is a chance that the microorganisms spread in the product contained in the conduit at a speed that is higher than the flow speed of the product. Thus the risk of contamination of an already sterilised product remains.

A way of achieving reliable killing of microorganisms would be, in principle, to heat the flow of products over an extremely long distance. However, such a device would require quite a large space.

### Summary of the Invention

The object of the present invention is thus to provide an improved device for reliable killing of microorganisms in a fluid flow of products and an improved corresponding method.

Above and in the following description killing of microorganisms means killing of such microorganisms that, for different reasons, are not desirable in the flow of products once sterilisation has been completed. Thus other microorganisms, which, for example, are not injurious to the health or do not affect the product durability and which require a higher temperature and/or a longer period of time to be killed, may be allowed to survive the sterilisation of the flow of products.

In order to achieve the object a device according to claim 1 as well as a method according to claim 10 are provided. Preferred embodiments of the device and the method are apparent from the dependent claims. The invention also relates to use of a device according to the invention according to claim 14.

More specifically, according to the present invention, a device is provided for killing microorganisms in a fluid flow of products, the device being characterised by a duct means made of a flexible material for conveying a flow of products heated to a predetermined temperature and a delimiting means comprising, for the purpose of delimiting non-intercommunicating subvolumes of the duct means and of transporting through the duct means a subquantity of the flow of products contained in each subvolume, pressing means that, alternately and in a starting position along the duct means, are adapted to engage with the duct means for compression thereof and, while maintaining said engagement, to move along a portion of the duct means, two succeding pressing means thus delimiting between them a subvolume that does not communicate with the rest of the volume of the duct means.

Thus a device is provided that ensures that a flow of products supplied to it and heated to a predetermined temperature is divided into separate subquantities, which during transport through the duct means are kept apart from each other. It is therefore not possible for the microorganisms to travel from one subquantity to another. Accordingly, once the subquantities are finally discharged from the device it is possible to ensure that they are sterile, which makes it possible to achieve a reliable sterilisation of the entire flow of products.

According to a preferred embodiment of the present invention, the device comprises a means for maintaining a relevant temperature in the heated flow of products. This means may, for example, ensure that an amount of steam is introduced into the subvolume with each subquantity of the flow of products, said steam maintaining the required temperature. Alternatively, said means may serve to insulate the device.

Preferably, the delimiting means is adapted to transport each subquantity during a period of time required for killing the microorganisms in each subquantity at the temperature concerned. Thus it becomes possible to ensure that each subquantity is retained at the relevant temperature during the time required for killing the microorganisms, whereby most reliable killing of the microorganisms is achieved.

Preferably, the subvolumes delimited by the delimiting means are constant in terms of volume. It will be appreciated, however, that the subquantities of the flow of products contained in the respective subvolumes need not be constant in terms of volume.

The delimiting means of the device is preferably adapted to connect the subvolume, after said transport of a subquantity of the flow of products contained in the respective subvolumes, with a portion of the duct means located downstream of the subvolume and to delimit a new subvolume. This results in a cyclically operating device that feeds the flow of products.

According to another preferred embodiment of the device, each pressing means is adapted to discontinue its engagement after said move and to return to said starting position. Here, the clamping means are preferably each supported by an arm arranged on a common rotatable shaft, and the duct means is preferably concentrically arranged around said shaft in a helical path, the subquantity of the flow of products contained in each subvolume being fed through the duct means by the rotation of the shaft. The shaft is advantageously arranged in the centre of a cylindrical housing, wherein the clamping means are adapted to compress the duct means against the inner wall of the housing.

The housing may be pressurised to provide a pressure outside the duct means similar to the one inside the duct means. This allows use of a duct means, which certainly is resistant to mechanical pressure, but which is less resistant to pressure differences between the inside and the outside of the duct means.

The duct means may be arranged in one, two or more turns, not necessarily complete ones, around said rotatable shaft.

The embodiments described above and the variants of the device according to the invention may, of course, be combined in an optional way.

According to the present invention, a device for killing microorganisms in a fluid flow of products is further provided, which is characterised by a duct means for conveying a flow of products heated to a predetermined temperature and a delimiting means adapted to delimit non-intercommunicating subvolumes of the duct means and to transport a subquantity of the flow of products contained in each subvolume through the duct means, means being arranged for maintaining the relevant temperature during said transport of the subquantities of the flow of products.

According to the present invention, a method for killing microorganisms in a fluid flow of products is further provided, which is characterised by the steps of heating the flow of products to a predetermined temperature, feeding the flow of products to a duct means made of a flexible material, engaging, alternately and in a starting position along the duct means, with the duct means for compression thereof by means of clamping means, two succeeding pressing means thus delimiting between them a subvolume that does not communicate with the rest of the volume of the duct means, and of transporting, by moving the pressing means along a portion of said duct means while maintaining said engagement, a subquantity of the flow of products contained in each subvolume, and thus separated from the rest of the flow of products, through the duct means while maintaining the relevant temperature.

Accordingly, a method is provided, which in a reliable way allows killing of microorganisms in the flow of products.

Preferably, a subquantity of the flow of products contained in each subvolume is transported through the duct means during the period of time required for killing the microorganisms in the respective subquantities at the temperature concerned.

Preferably, each delimited subvolume is essentially constant in terms of volume.

Preferably, the method further comprises the step of feeding a steam flow to the duct means together with the flow of products, whereby each subvolume is filled on the one hand with a subquantity of the flow of products and, on the other hand, with a subquantity of the steam flow. In this way, it is easy to ensure that the required temperature.is maintained in the flow of products.

According to the present invention, a method for killing microorganisms in a fluid flow of products is further provided, which is characterised by the steps of heating the flow of products to a predetermined temperature, feeding the flow of products to a duct means, delimiting non-intercommunicating subvolumes of the duct means, and transporting a subquantity of the flow of products contained in each subvolume, and thus separated from the rest of the flow of products, through the duct means while maintaining the relevant temperature.

Finally, also use of a device as defined above is provided according to the invention.

The present invention will now be described for exemplifying purposes with reference to the accompanying drawings.

### Brief Description of the Drawings

Fig. 1 is a side view of an embodiment of the device according to the present invention.
Fig. 2 is a partly sectioned side view of the device shown in Fig. 1.
Fig. 3 is a top plan view of the device in Fig. 1, where a lid of the device has been removed.

### Description of Embodiments

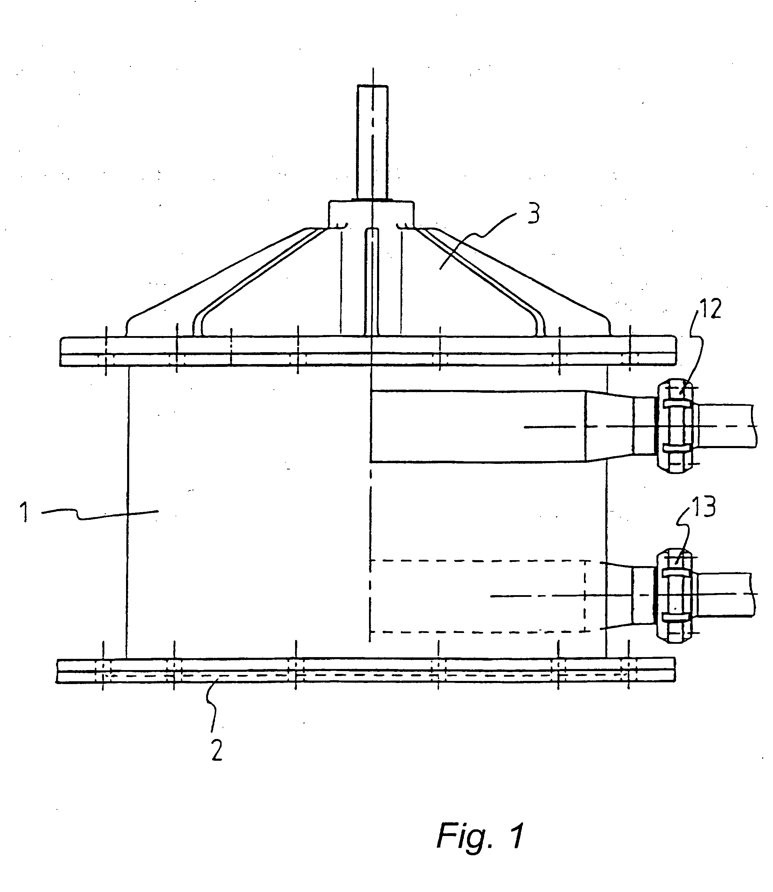

With reference to Figs 1-3, a device is illustrated for killing microorganisms according to a preferred embodiment of the present invention.

The device comprises a cylindrical housing 1, the bottom end of which is sealed by means of a bottom plate 2 and the top end of which is sealed by means of a lid 3.

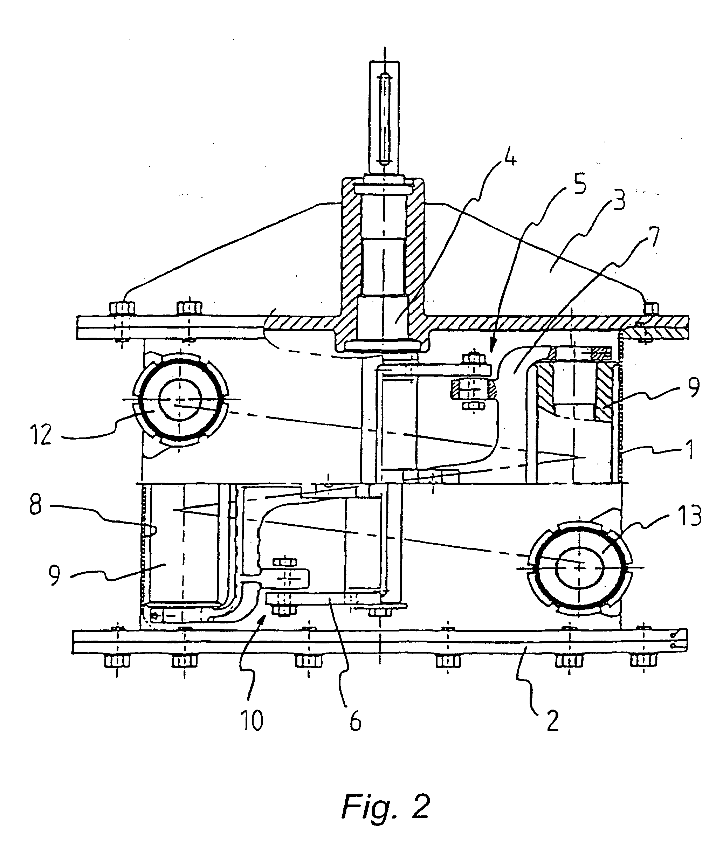

Inside the housing, a shaft 4 is arranged extending along the longitudinal centre axis of the housing 1. The shaft 4 is rotatably mounted in the lid 3 of the housing 1. The shaft 4 supports a delimiting means 5, which comprises four holding means 6 evenly distributed around the circumference. In each holding means 6 an arm 7 is mounted, which extends towards the inner wall 8 of the housing 1. Each arm 7 supports, in turn, a pressing means in the form of a roller 9 extending parallel to the inner wall 8. As shown in Fig. 2, the rollers 9 have a length that essentially corresponds to the height of the cylindrical housing 1. Each arm 7 comprises a fixing means 10 allowing adjustment of the bearing pressure of the roller 9 against the inner wall 8. The shaft 4 is rotatable with the aid of a driving means (not shown), whereby the rollers 9 are rotatable within the housing 1 along its inner wall 8.

Furthermore, a duct means in the form of a tube 11 is concentrically arranged around the shaft 4, thus extending along the inner wall 8 of the housing 1, between said wall 8 and the rollers 9. The tube 11 is made of a flexible material and extends one and a half turns in a helical/spiralling path around the shaft 4 between an inlet 12 and an outlet 13, which is indicated in Fig. 2 by a dash-dotted line between the inlet 12 and the outlet 13. Naturally, the number of turns by which the tube 11 extends around said shaft 4 is not limited to the number shown. Consequently, both a greater and a smaller number of turns, not necessarily complete ones, are conceivable. The inlet 12 and the outlet 13 connect tangentially, on opposite sides, to the housing 1 and are oriented the same way and vertically offset.

Connecting conduits 14,15 are connected to the inlet 12 and the outlet 13.

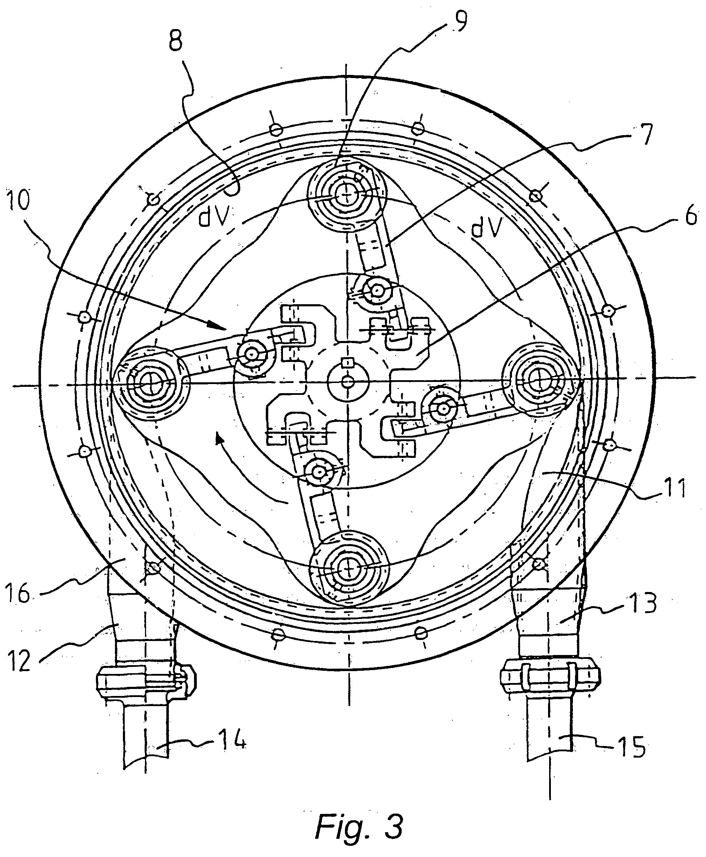

When using the device according to the invention a heated and pressurised fluid flow of products is supplied to the device through one of the connecting conduits 14. Said heating may, for example, be effected by supplying the flow of products in the form of drops and/or film to a volume filled with steam. More specifically, the heated flow of products is fed to the tube 11 of the device through the inlet 12. The shaft 4 is rotated in the direction indicated by the arrow in Fig. 3.

During the rotation of the shaft 4, the rollers 9 will be moved along the tube 11. Furthermore, the engagement of each roller 9 with the tube 11 will result in a compression of the tube 11. Consequently, two subsequent rollers 9 will delimit between them a subvolume dV separated from the remaining volume of the tube 11. Thus the subvolumes dV delimited by the engagement of the rollers 9 with the tube 11 cannot communicate with each other.

The subvolumes dV formed by the engagement of the rollers 9 with the tube 11 are essentially constant in terms of volume. It will be appreciated, however, that the subquantities of the flow of products need not be constant in terms of volume.

The device may further comprise a means for maintaining the relevant temperature in the subquantities during their transport through the duct means. Such a means may, for example, be adapted to introduce steam into the respective subvolumes. Thus each subquantity of the flow of products need not fill up its subvolume dV. Accordingly, it is conceivable - as well as preferred - that each subquantity only fills up part of its subvolume dV and that steam, preferably at the same positive pressure as the flow of products, fills up the rest of the subvolume dV. The pressure and temperature of the steam ensure that the required temperature can be maintained in each subquantity of the flow of products while being fed through the device. In the above described case, where the flow of products is heated by introducing it in the form of drops and/or a film to a volume filled with steam, the required temperature can thereby be ensured by letting a part of the heating medium, i.e. the steam, accompany the flow of products to the device according to the invention at maintained pressure and temperature.

The flow of products that enters through the inlet 12 will thus be continuously supplied to the non-intercommunicating subvolumes dV of the tube 11 and thereby divided into separate subquantities. These subquantities will then be passed on by the rotation of the shaft 4, while being kept apart from each other. A product film may indeed form on the inside of the tube 11 and get in contact with the different subquantities. However, any product film formed will remain in the device for such a long time that the film will become sterile, and therefore there is no risk of contamination of the different subquantities.

Each subvolume dV and the subquantity of the flow of products contained therein will be passed on through the action of two subsequent rollers 9 so that they, after one and a half turns, may be supplied to the tube segment or part 16 of the tube 11 that leads to the outlet 13 of the device. The front one of the two rollers 9, in the direction of rotation, will by its rotation first connect the subvolume dV with the tube segment 16 downstream of the subvolume dV, whereupon the rear roller 9, during its continued rotation, will press out the subquantity of the flow of products concerned through the tube segment 16 concerned. The same procedure will then be repeated for the following subvolumes dV and the subquantities of the flow of products contained therein during the continued rotation of the shaft 4.

As mentioned previously, the flow of products has been heated when it is fed to the device. In order to achieve the desired killing of microorganisms, each subquantity must be maintained at a predetermined temperature for a specific period of time. Accordingly, the killing is a function of time and temperature. This function means, for example, that a subquantity with a lower temperature must be kept at this temperature for a longer period of time.

For this purpose, the shaft 4 of the device is adapted to rotate at a speed that is adjusted to the temperature of the incoming flow of products as well as to the distance that each subvolume dV and the subquantity contained therein is moved. This makes it possible to ensure that each subquantity of the flow of products is maintained at the required temperature during the required period of time. In particular, it is possible to ensure that each subquantity is maintained at an absolute, controlled temperature during a required period of time without the risk of local overheating or the risk of too long holding times locally.

In this context, it should be mentioned that the temperature and time need not be set so that all microorganisms present in the respective subquantities are killed. It may, for example, be enough to ensure that all health-impairing and product-damaging microorganisms are killed. It is also possible that there may be harmless microorganisms, which survive the processing. Consequently, what is important is that time and temperature are adjusted so that a number of pre-selected types of microorganisms are killed.

As mentioned previously, the duct means is preferably composed of a tube 11 made of a flexible material, preferably a plastic material. Such a tube 11 presents several advantages.

The tube 11 is particularly easy to clean. This is done by the introduction of a cleaning agent into the device, which is operated as usual. The lack of corners, gaps and the like in the tube 11 ensures an excellent cleaning result.

Furthermore, the tube 11 is easily replaced. This can be done when the tube 11 is worn-out or as an alternative to cleaning the tube 11.

The tube 11 being made of a plastic material it does not have the same burning effect as, for example, a stainless steel pipe that is heated to the same temperature. Thus, if the flow of products consists of milk, the problem associated with steel pipes, i.e. the milk being burnt when it makes contact with the hot inner wall of the pipe, is avoided.

Certain flexible plastic materials resist mechanical pressure well, whereas their resistibility to pressure differences is limited. There is therefore a risk that a tube 11 made of such a material will be exposed to great stress, since the flow of products is normally introduced into the tube 11 at a pressure of 2-5 bars and the pressure inside the device, on the outside of the tube 11, is either atmospheric or slightly negative. To solve this problem the housing 1 of the device is preferably pressurised in order to obtain a similar pressure on the inside and outside of the tube 11. By ensuring that the pressure on the outside of the tube 11 is constant, alterations of the shape of the tube 11 caused by pressure differences are avoided, which in turn ensures that the subvolumes dV formed by the engagement of the rollers 9 with the tube 11 remain essentially constant in terms of volume.

As mentioned above the flow of products fed to the device is preferably pressurised. This allows the flow of products to be kept at a sufficiently high temperature without boiling. Moreover, the device works advantageously against an atmospheric pressure or a slightly negative pressure, which, for example, may mean that the outlet 13 is connected to a tank (not shown) with atmospheric pressure. Once a connection is established between a subvolume dV and the tube segment 16, which in turn is connected with the outlet 13, the subquantity of the flow of products contained in the subvolume dV will be sucked out of the subvolume dV, through the outlet 13 and into the tank. Accordingly, a fast and reliable evacuation of the subvolumes dV is achieved.

According to the invention, a device is thus provided that allows reliable killing of microorganisms. More specifically, this is achieved by dividing the flow of products into separate subquantities, which are then kept apart during a period of time that, depending on the temperature of the flow of products, is sufficient to ensure that the desired microorganisms are killed. The risk of the microorganisms spreading in the flow of products and contaminating an already sterilised product is thereby eliminated.

The scope of the invention is defined solely by the appended claims.

## Claims

1. A device for killing microorganisms in a fluid flow of products,
**characterised by**
a duct means (11) made of a flexible material for conveying a flow of products heated to a predetermined temperature, and
a delimiting means (5) comprising, for delimiting non-intercommunicating subvolumes (dV) of the duct means (11) and for transporting through the duct means (11) a subquantity of the flow of products contained in each subvolume (dV), pressing means (9) which,
alternately and in a starting position along the duct means (11), are adapted to engage with the duct means (11) for compression thereof and which,
while maintaining said engagement, are adapted to move along a portion of the duct means (11),
whereby two succeeding pressing means (9) delimit between them a subvolume (dV) that does not communicate with the rest of the volume of the duct means (dv) wherein the device comprises a means for maintaining a relevant temperature in the heated flow of products.

2. A device according to claim 1, wherein the delimiting means (5) is adapted to transport each subquantity during the period of time required for killing the microorganisms in the respective subquantity at the temperature concerned.

3. A device according to claim 1 or 2, wherein delimited subvolumes (dV) delimited by the delimiting means (5) are constant in terms of volume.

4. A device according to any one of the preceding claims, wherein the delimiting means (5), after said transport of a subquantity of the flow of products contained in the respective subvolume, is adapted to connect the subvolume (dV) with a portion (16) of the duct means (11) located downstream of the subvolume (dV) and to delimit a new subvolume (dV).

5. A device according to any one of the preceding claims, in which each pressing means (9) is adapted to discontinue its engagement after said move and to return to said starting position.

6. A device according to any one of the preceding claims, in which each pressing means (9) is supported by an arm (7) arranged on a common rotatable shaft (4) and the duct means (11) is concentrically arranged around said shaft (4) in a helical path, the subquantity of the flow of products contained in each subvolume (dV) being fed through the duct means (11) by the rotation of the shaft (4).

7. A device according to claim 6, in which said shaft (4) is arranged in the centre of a cylindrical housing (1), the pressing means being adapted to compress the duct means (11) against the inner wall (8) of the housing (1).

8. A device according to claim 7, wherein the housing (1) is pressurised to provide a pressure outside the duct means (11) similar to the one inside the duct means (11).

9. A device according to claims 6-8, wherein the duct means (11) is arranged in one, two or more turns, not necessarily complete ones, around said rotatable shaft (4).

10. A method for killing microorganisms in a fluid flow of products, **characterised by** the steps of
heating the flow of products to a predetermined temperature,
feeding the flow of products to a duct means (11) made of a flexible material,
engaging, alternately and at a starting position along the duct means (11), with the duct means (11) for compression thereof by means of pressing means (9), two succeeding pressing means (9) thus delimiting between them a subvolume (dV) that does not communicate with the rest of the volume of the duct means (dV), and
transporting, by moving the pressing means (9) along a portion of the duct means (11) while maintaining said engagement, a subquantity of the flow of products contained in each subvolume (dV), and thus separated from the rest of the flow of products, through the duct means (11) while maintaining the relevant temperature.

11. A method according to claim 10, wherein a subquantity of the flow of products contained in each subvolume (dV) is transported through the duct means (11) during the period of time required for killing the microorganisms in the respective subquantity at the temperature concerned.

12. A method according to any one of claims 10-11, wherein each delimited subvolume (dV) is constant in terms of volume.

13. A method according to any one of claims 10-12 further comprising the step of feeding a steam flow to the duct means (11) together with the flow of products, whereby the respective subvolumes (dV) are filled on the one hand with a subquantity of the flow of products and, on the other, with a subquantity of the steam flow.

14. The use of a device according to any one of claims 1-9 for killing microorganisms in a fluid flow of products.

## Patentansprüche

1. Vorrichtung zum Abtöten von Mikroorganismen in einem Fluidstrom von Erzeugnissen, **gekennzeichnet durch**:
eine Leitungseinrichtung (11), die aus einem flexiblen Material besteht und einen Strom von Erzeugnissen befördert, der auf eine vorgegebene Temperatur erhitzt ist, und
eine Abgrenzeinrichtung (5), die zum Abgrenzen nicht miteinander kommunizierender Teilvolumina (dV) der Leitungseinrichtung (11) und zum Transportieren einer Teilmenge des Stroms von Erzeugnissen, die in jedem Teilvolumen (dV) enthalten ist, **durch** die Leitungseinrichtung (11) Presseinrichtungen (9) umfasst, die:
so eingerichtet sind, dass sie abwechselnd und in einer Ausgangsposition entlang der Leitungseinrichtung (11) mit der Leitungseinrichtung (11) in Eingriff kommen, um diese zusammenzudrücken, und die:
so eingerichtet sind, dass sie sich, während sie den Eingriff aufrechterhält, an einem Abschnitt der Leitungseinrichtung (11) entlangbewegen,
wobei zwischen zwei aufeinanderfolgenden Presseinrichtungen (9) ein Teilvolumen (dV) abgegrenzt wird, das mit dem Rest des Volumens der Leitungseinrichtung (dV) nicht kommuniziert, und wobei die Vorrichtung eine Einrichtung zum Aufrechterhalten einer entsprechenden Temperatur in dem erhitzten Strom von Erzeugnissen umfasst.

2. Vorrichtung nach Anspruch 1, wobei die Abgrenzeinrichtung (5) so eingerichtet ist, dass sie jede Teilmenge während des Zeitraums, der zum Abtöten der Mikroorganismen in der jeweiligen Teilmenge erforderlich ist, bei der betreffenden Temperatur transportiert.

3. Vorrichtung nach Anspruch 1 oder 2, wobei abgegrenzte Teilvolumina (dV), die durch die Abgrenzeinrichtung (5) abgegrenzt werden, hinsichtlich des Volumens konstant sind.

4. Vorrichtung nach einem der vorangehenden Ansprüche, wobei die Abgrenzeinrichtung (5) so eingerichtet ist, dass sie nach dem Transport einer Teilmenge des Stroms von Erzeugnissen, die in dem jeweiligen Teilvolumen enthalten ist, das Teilvolumen (dV) mit einem Abschnitt (16) der Leitungseinrichtung (11) verbindet, der sich vor dem Teilvolumen (dV) befindet, und ein neues Teilvolumen (dV) abgrenzt.

5. Vorrichtung nach einem der vorangehenden Ansprüche, wobei jede Presseinrichtung (9) so eingerichtet ist, dass sie ihren Eingriff nach der Bewegung unterbricht und an die Ausgangsposition zurückkehrt.

6. Vorrichtung nach einem der vorangehenden Ansprüche, wobei jede Presseinrichtung (9) von einem Arm (7) getragen wird, der an einer gemeinsamen drehbaren Welle (4) angeordnet ist und die Leitungseinrichtung (11) auf einem schraubenförmigen Weg konzentrisch um die Welle (4) herum angeordnet ist, wobei die Teilmenge des Stroms von Erzeugnissen, die in jedem Teilvolumen (dV) enthalten ist, durch die Drehung der Welle (4) durch die Leitungseinrichtung (11) hindurch geleitet wird.

7. Vorrichtung nach Anspruch 6, wobei die Welle (4) in der Mitte eines zylindrischen Gehäuses (1) angeordnet ist und die Presseinrichtungen so eingerichtet sind, dass sie die Leitungseinrichtung (11) an die Innenwand (8) des Gehäuses (1) zusammendrücken.

8. Vorrichtung nach Anspruch 7, wobei das Gehäuse (1) unter.Druck gesetzt wird, um einen Druck außerhalb der Leitungseinrichtung (11) zu erzeugen, der dem innerhalb der Leitungseinrichtung (11) gleicht.

9. Vorrichtung nach den Ansprüchen 6-8, wobei die Leitungseinrichtung (11) in einer, zwei oder mehreren Windungen um die drehbare Welle (4) herum angeordnet ist, die nicht notwendigerweise vollständige Windungen sind.

10. Verfahren zum Abtöten von Mikroorganismen in einem Fluidstrom von Erzeugnissen, **gekennzeichnet durch** die folgenden Schritte:
Erhitzen des Stroms von Erzeugnissen auf eine vorgegebene Temperatur,
Leiten des Stroms von Erzeugnissen zu einer Leitungseinrichtung (11 ), die aus einem flexiblen Material besteht,
Herstellen von Eingriff der Leitungseinrichtung (11) abwechselnd und an einer Ausgangsposition entlang der Leitungseinrichtung (11) mit zwei aufeinanderfolgenden Presseinrichtungen (9) zum Zusammendrücken derselben mittels der Presseinrichtungen (9), um so zwischen ihnen ein Teilvolumen (dV) abzugrenzen, das nicht mit dem Rest des Volumens der Leitungseinrichtung (dV) kommuniziert, und
Transportieren einer Teilmenge des Stroms von Erzeugnissen, die in jedem Teilvolumen (dV) enthalten ist und so vom Rest des Stroms von Erzeugnissen getrennt ist, **durch** die Leitungseinrichtung (11) bei Aufrechterhaltung der entsprechenden Temperatur, **durch** Bewegen der Presseinrichtung (9) entlang eines Abschnitts der Leitungseinrichtung (11) bei Aufrechterhaltung des Eingriffs.

11. Verfahren nach Anspruch 10, wobei eine Teilmenge des Stroms von Erzeugnissen, die in jedem Teilvolumen (dV) enthalten ist, während des Zeitraums, der zum Abtöten der Mikroorganismen in der jeweiligen Teilmenge erforderlich ist, bei der betreffenden Temperatur durch die Leitungseinrichtung (11) transportiert wird.

12. Verfahren nach einem der Ansprüche 10-11, wobei jedes abgegrenzte Teilvolumen (dV) hinsichtlich des Volumens konstant ist.

13. Verfahren nach einem der Ansprüche 10-12, das des weiteren den Schritt des Leitens eines Dampfstroms zu der Leitungseinrichtung (11) zusammen mit dem Strom von Erzeugnissen umfasst, wobei die jeweiligen Teilvolumina (dV) einerseits mit einer Teilmenge des Stroms von Erzeugnissen und andererseits mit einer Teilmenge des Dampfstroms gefüllt werden.

14. Einsatz einer Vorrichtung nach einem der Ansprüche 1-9 zum Abtöten von Mikroorganismen in einem Fluidstrom von Erzeugnissen.

## Revendications

1. Dispositif pour tuer des microorganismes dans un écoulement fluide de produits,
**caractérisé par**
un moyen de conduit (11) composé d'un matériau flexible pour transporter un écoulement de produits chauffé à une température prédéterminée, et
un moyen de délimitation (5) comprenant, pour délimiter les sous-volumes (dV) qui ne sont pas intercommunicants du moyen de conduit (11) et pour transporter au travers du moyen de conduit (11) une sous-quantité de l'écoulement de produits contenue dans chaque sous-volume (dV) , des moyens de presse (9) qui,
de manière alternée et dans une position de départ le long du moyen de conduit (11), sont adaptés pour être engagés avec le moyen de conduit pour la compression de celui-ci et qui,
tout en maintenant ledit engagement, sont adaptés pour se déplacer le long d'une partie du moyen de conduit (11)
de sorte que deux moyens de presse successifs (9) délimitent entre eux un sous-volume (dV) qui ne communique pas avec le reste du volume du moyen de conduit (dV) où le dispositif comprend un moyen pour maintenir une température pertinente dans l'écoulement de produits chauffé.

2. Dispositif selon la revendication 1, dans lequel le moyen de délimitation (5) est adapté pour transporter chaque sous-quantité au cours de la période de temps nécessaire pour tuer les microorganismes dans la sous-quantité respective à la température concernée.

3. Dispositif selon la revendication 1 ou 2, dans lequel les sous-volumes délimités (dV), délimités par le moyen de délimitation (5) sont constants en terme de volume.

4. Dispositif selon l'une quelconque des revendications précédentes, dans lequel le moyen de délimitation (5), après ledit transport d'une sous-quantité de l'écoulement de produits contenue dans le sous-volume respectif, est adapté pour relier le sous-volume (dV) avec une partie (16) du moyen de conduit (11) situé en aval du sous-volume (dV) et pour délimiter un nouveau sous-volume (dV).

5. Dispositif selon l'une quelconque des revendications précédentes, dans lequel chaque moyen de presse (9) est adapté pour interrompre son engagement après ledit déplacement et pour retourner à ladite position de départ.

6. Dispositif selon l'une quelconque des revendications précédentes, dans lequel chaque moyen de presse (9) est soutenu par un bras (7) disposé sur une tige rotative courante (4) et le moyen de conduit (11) est disposé de manière concentrique autour de ladite tige (4) selon une trajectoire hélicoïdale, la sous-quantité de l'écoulement de produit contenue dans chaque sous-volume (dV) étant alimentée au travers du moyen de conduit (11) par la rotation de la tige (4).

7. Dispositif selon la revendication 6, dans lequel ladite tige (4) est disposée dans le centre d'un boîtier cylindrique (1), le moyen de presse étant adapté pour comprimer le moyen de conduit (11) contre la paroi interne (8) du boîtier (1).

8. Dispositif selon la revendication 7, dans lequel le boîtier (1) est pressurisé pour fournir une pression à l'extérieur du moyen de conduit (11) similaire à celle à l'intérieur du moyen de conduit (11).

9. Dispositif selon les revendications 6 à 8, dans lequel le moyen de conduit (11) est disposé en un, deux tours ou plus, pas nécessairement des tours complets, autour de ladite tige rotative (4).

10. Procédé pour tuer les microorganismes dans un écoulement de produits, **caractérisé par** les étapes consistant à :
chauffer l'écoulement de produit à une température prédéterminée,
alimenter l'écoulement de produits à un moyen de conduit (11) composé d'un matériau flexible,
engager, de manière alternée et à une position de départ le long du moyen de conduit (11), avec le moyen de conduit (11) pour la compression de celui-ci par des moyens de presse (9), deux moyens de presse successifs (9) délimitant ainsi entre eux un sous-volume (dV) qui ne communique pas avec le reste du volume du moyen de conduit (dV) et
transporter, en déplaçant les moyens de presse (9) le long d'une partie du moyen de conduit (11) tout en maintenant ledit engagement, une sous-quantité de l'écoulement de produits contenue dans chaque sous-volume (dV), et ainsi séparée du reste de l'écoulement de produits, au travers du moyen de conduit (11) tout en maintenant la température pertinente.

11. Procédé selon la revendication 10, dans lequel une sous-quantité de l'écoulement de produits contenu dans chaque sous-volume (dV) est transportée au travers du moyen de conduit (11) au cours de la période de temps nécessaire pour tuer les microorganismes dans la sous-quantité respective à la température concernée.

12. Procédé selon l'une quelconque des revendications 10 à 11, dans lequel chaque sous-volume délimité (dV) est constant en terme de volume.

13. Procédé selon l'une quelconque des revendications 10 à 12 comprenant en outre l'étape consistant à alimenter un écoulement de vapeur au moyen de conduit (11) conjointement avec l'écoulement de produits, de sorte que les sous-volumes respectifs (dV) soient chargés d'un côté avec une sous-quantité de l'écoulement de produits et de l'autre, avec une sous-quantité de l'écoulement de vapeur.

14. Utilisation d'un dispositif selon l'une quelconque des revendications 1 à 9 pour tuer des microorganismes dans un écoulement fluide de produits.
